# EUROPEAN PATENT SPECIFICATION

(11) **EP 4 183 450 B1**
(45) Date of publication and mention of the grant of the patent: **20.08.2025**
(21) Application number: 21215144.3
(22) Date of filing: 16.12.2021
(51) Int. Cl.: C07D 209/42

(54) **METHOD FOR PREPARING A HYDRATED FORM OF PERINDOPRIL L-ARGININE**
VERFAHREN ZUR HERSTELLUNG EINER HYDRATISIERTEN FORM VON PERINDOPRIL L-ARGININ
PROCÉDÉ DE PRÉPARATION D'UNE FORME HYDRATÉE DE PÉRINDOPRIL L-ARGININE

(30) Priority: 18.11.2021 SI 202100204
(43) Date of publication of application: 24.05.2023
(73) Proprietor: Zupet, Rok, 1211 Ljubljana - Smartno (SI)
(72) Inventor: Zupet, Rok, 1211 Ljubljana -Smartno (SI); Zupancic, Blaz, 1000 Ljubljana (SI)
(74) Representative: Redensek, Vladimira

(56) References cited:
- CN-A- 110 283 104
- US-A1- 2011 301 357

## Description

### Field of invention

The present invention relates to a novel process for preparing a hydrated form of perindopril L-arginine, characterised by short filtration times, high molar yield, high chromatographic purity and adequate stability of the final product perindopril L-arginine at room temperature.

### Subject of invention

The present invention relates to a novel process for preparing a hydrated form of perindopril L-arginine with improved process properties.

The process according to the invention includes a crystallisation process for the formation of a product, a hydrated form of perindopril L-arginine, wherein a suspension of the hydrated form of perindopril L-arginine formed in one of the steps of the process has excellent processing properties, which is achieved by precisely controlling and regulating the amount of water present in the process. The formed suspension of the hydrated form of perindopril L-arginine is stable and has a short filtration time, and the process according to the invention at the same time has a high molar yield comparable to the yields of the processes known in the prior art. The product obtained has high chromatographic purity and adequate stability under storage conditions at room temperature.

The process according to the invention is transferable to a large scale and is suitable for use on an industrial scale and provides an efficient, economical and reproducible process for the production of a hydrated form of perindopril L-arginine using materials that can be readily regenerated with a minimal environmental footprint.

### Technical problem and prior art

Angiotensin-converting enzyme inhibitors (ACE inhibitors) are used to treat high blood pressure and certain types of cardiovascular diseases. Perindopril, with the chemical name (2S)-2-[(1S)-1-carbethoxybutylamino]-1-oxopropyl-(2S, 3aS, 7aS)-perhydroindole-2-carboxylic acid, is a potent ACE inhibitor.

The prior art literature contains several descriptions of anhydrous and hydrated forms of perindopril L-arginine and of a process for preparing perindopril L-arginine, but nowhere is there a description of a process with a high molar yield and an industrially applicable process with good isolation properties for the production of a hydrated form of perindopril L-arginine.

The anhydrous forms of perindopril L-arginine (beta and delta) can be produced in high yield and with good filtration properties. The hydrated polymorphic forms (alpha, gamma) of perindopril L-arginine can also be produced in high yield, but always with slower filtration of the product, which makes efficient and rapid production on an industrial scale difficult. For example, when rapid filtration of a suspension of the product is described and when the amount of water in the suspension is lower than the molar equivalent based on the amount of peridopril L-arginine, the observed molar yields of the crystallisation processes are significantly lower. The prior art literature does not describe a reproducible crystallisation process for preparing a hydrated form of perindopril L-arginine with high molar yield and good filtration properties.

US 4,508,729 describes perindopril and its pharmaceutically acceptable salts, where a pharmaceutically acceptable salt is selected from the group consisting of salts of mineral or organic acids or bases, such as sodium salt or maleate salt.

Perindopril L-arginine, its hydrates, a pharmaceutical composition and a treatment process were first described in patent US 6,696,481 B2.

The alpha and beta forms of perindopril L-arginine are described in patent documents EP 1989182 B1 and EP 2016051 B1. The forms were prepared by crystallisation from a mixture of several solvents.

The gamma hydrated form of perindopril L-arginine is described in patent EP 2682388 B1, where it was produced by spray-drying, freeze-drying, suspension or various crystallisation processes. In cases where high molar yields of the process are reported, the isolation properties of the suspension are poor - long filtration times of the product, the hydrated form of perindopril L-arginine.

Improved isolation properties of the crystallisation process are described in patent application CN 112047999 A. In the examples 1-4 described herein, the molar yield of conversion to perindopril L-arginine from perindopril tert-butylamine was 68-81 %.

High molar yields of conversion from perindopril tert-butylamine to perindopril L-arginine are described in patent application CN 110283104 A.

High molar yields of the crystallisation process of perindopril L-arginine gamma form with rapid filtration properties are described in patent application CN 103172696 A.

The delta form of perindopril L-arginine is described in patent EP 2612850 B1, where the filtration properties of the isolated product were investigated in detail. The thermal stability of the product under stress conditions has been studied and described in detail. The delta form has been shown to be more stable under stress conditions compared to the hydrated or amorphous form.

### Description of the solution to the technical problem with examples

There is a market need for a process for preparing a hydrated form of perindopril L-arginine with a high molar yield that is cost-competitive and that can be readily used in multi-purpose production equipment.

In the context of the invention, the hydrated form of perindopril L-arginine is understood to mean the hydrated polymorphic form of perindopril L-arginine.

It has been found that in the process for the production of a hydrated form of perindopril L-arginine, by controlling and regulating the amount of water in the crystallisation solution comprising perindopril L-arginine, the isolation properties of the suspension of the hydrated form of perindopril L-arginine formed in the process are improved, i.e. the stability time of the obtained suspension is prolonged, the filtration time of the suspension is shortened and the amount of solvent and antisolvent in the final product - an isolated wet filter cake of the hydrated form of perindopril L-arginine - is reduced, wherein the wet filter cake has a high dry residue value after drying and wherein the hydrated form of perindopril L-arginine with a high molar yield is obtained.

The term 'dry residue after drying', expressed as a %, as used throughout the present patent application, is defined as the % (*w*/*w*) of the dry fraction of the product of the hydrated form of perindopril L-arginine in the total weight of the isolated wet filter cake. The term 'paste strength' is also used in the art, e.g. as described in EP 1539711 B1.

The term relative mother liquor flow rate, as used throughout the present patent application to estimate the filtration rate of the hydrated form of perindopril L-arginine, is defined as the mass flow rate of solvent and antisolvent per unit area (kg/h/m²). This parameter quantifies the filtration rate of a suspension of the hydrated form of perindopril L-arginine.

The molar yield of the process according to the present invention, expressed as a %, is understood as the molar yield of conversion from perindopril tert-butylamine or perindopril to the hydrated form of perindopril L-arginine.

In the process for preparing a hydrated form of perindopril L-arginine according to the invention, perindopril or a perindopril salt may be used as a starting material. Preferably perindopril or perindopril tert-butylamine is used as a starting material.

The subject of the present invention is therefore a novel process for preparing a hydrated form of perindopril L-arginine, wherein perindopril or a perindopril salt is used as a starting material and wherein the process comprises:
- forming a crystallisation solution comprising perindopril L-arginine, wherein forming the crystallisation solution comprises at least:
   - forming a perindopril solution, wherein ethanol is used as a solvent and ethyl acetate is used as an antisolvent, and wherein the perindopril solution formed contains less than 0.5 mol. equiv. of water with respect to perindopril,
   - adding L-arginine to the perindopril solution,
   to form a crystallisation solution comprising perindopril L-arginine,
- measuring the amount of water in the obtained crystallisation solution comprising perindopril L-arginine,
- adjusting the amount of water in the obtained crystallisation solution by adding such an amount of water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a suspension of the hydrated form of perindopril L-arginine, having a relative mother liquor flow rate in the range of 5500-6500 kg/h/m²,
- filtering the obtained suspension of the hydrated form of perindopril L-arginine,
to obtain a product, a wet filter cake of the hydrated form of perindopril L-arginine, having a dry residue value after drying in the range of 50-70%.

The process according to the invention is characterised by a high molar yield, the molar yield of the process being 85-92%, preferably the molar yield of the process being 87-92%.

The suspension formed in the step of forming a suspension of the hydrated form of perindopril L-arginine has good filtration properties and has a relative mother liquor flow rate in the range of 5500-6500 kg/h/m².

In one embodiment, the present invention relates to a process for preparing a hydrated form of perindopril L-arginine, wherein perindopril tert-butylamine is used as a starting material and wherein the process comprises:
- forming a crystallisation solution comprising perindopril L-arginine, wherein forming the crystallisation solution comprises:
   - forming a perindopril solution, wherein the perindopril solution formed contains less than 0.5 mol. equiv. of water with respect to perindopril, comprising:
      - disolving perindopril tert-butylamine in a solvent, preferably water and dichloromethane,
      - adjusting the pH of the solution to a pH in the range of 3.8-4.2,
      - separating the resulting phases and washing the aqueous phase with dichloromethane,
      - combining the organic phases and adding a solvent ethanol to the organic phase,
      - distilling off the mixture of solvents to a water content in the perindopril solution of less than 0.5 mol. equiv. of water with respect to perindopril,
      - if needed, adding a solvent ethanol, and distilling off the mixture of solvents until a water content in the perindopril solution of less than 0.5 mol. equiv. of water with respect to perindopril is achieved,
   - adding L-arginine in an amount of up to 6% of the molar excess of L-arginine, preferably 2-4% of the molar excess of L-arginine, with respect to perindopril,
   - distilling the obtained perindopril L-arginine solution to partially remove the solvent,
   - adding an antisolvent ethyl acetate,
   to form a crystallisation solution comprising perindopril L-arginine,
and then:
- measuring the amount of water in the obtained crystallisation solution comprising perindopril L-arginine,
- adjusting the amount of water in the obtained crystallisation solution by adding such an amount water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a stable suspension of the hydrated form of perindopril L-arginine, having a relative mother liquor flow rate in the range of 5500-6500 kg/h/m², and
- filtering a well-filterable suspension of the hydrated form of perindopril L-arginine, wherein the relative mother liquor flows are in the range of 5500-6500 kg/h/m²,

to obtain a product, a wet filter cake of the hydrated form of perindopril L-arginine, having a dry residue value after drying in the range of 50-70%
and the molar yield of the process after drying is 85-92%.

In a further embodiment, the present invention relates to a process for preparing a hydrated form of perindopril L-arginine, wherein perindopril is used as a starting material and wherein the process comprises:
- forming a crystallisation solution comprising perindopril L-arginine, wherein forming the crystallisation solution comprises:
   - forming a perindopril solution by dissolving perindopril in the solvent ethanol, wherein the perindopril solution formed contains less than 0.5 mol. equiv. of water with respect to perindopril,
   - adding L-arginine to the obtained solution in an amount of up to 6% of the molar excess of L-arginine, preferably 2-4% of the molar excess of L-arginine, with respect to perindopril,
   - distilling the obtained perindopril L-arginine solution to partially remove the solvent,
   - adding an antisolvent ethyl acetate,
   to form a crystallisation solution comprising perindopril L-arginine,
and then:
- measuring the amount of water in the obtained crystallisation solution comprising perindopril L-arginine,
- adjusting the amount of water in the obtained crystallisation solution by adding such an amount of water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a stable suspension comprising a hydrated form of perindopril L-arginine, having a relative mother liquor flow rate in the range of 5500-6500 kg/h/m², and
- filtering the obtained suspension, wherein the relative mother liquor flow is in the range of 5500-6500 kg/h/m²,
to obtain a product, a wet filter cake of the hydrated form of perindopril L-arginine, having a dry residue value after drying in the range of 50-70% and the molar yield of the process after drying is 85-92%.

A yet further embodiment of the process according to the invention is a preparation of a hydrated form of perindopril L-arginine, from perindopril as a starting material, wherein perindopril is dissolved in ethanol at 45°C. L-arginine is added to this solution and stirred at 45°C for 10-15 minutes or until a clear solution is formed. The solvent is partially removed using techniques such as vacuum or atmospheric distillation. To the resulting clear solution, an antisolvent ethyl acetate at room temperature, is added, then the crystallisation solution is seeded while stirred and the amount of water is adjusted to give a suspension of the hydrated form of perindopril L-arginine, which is filtered and dried.

In the process according to the invention ethyl acetate isused as an antisolvent.

The amount of water in the resulting crystallisation solution is controlled and adjusted by adding such an amount of water to the crystallisation solution that the amount of water in the crystallisation solution is adjusted to 1.0-1.4 molar equivalents of water, calculated with respect to perindopril L-arginine, preferably 1.1-1.3 molar equivalents of water.

Less than 1.0 molar equivalent of water, calculated with respect to perindopril L-arginine, in the crystallisation solution results in a lower yield of the hydrated form of perindopril L-arginine.

Prior art processes for the crystallisation of the hydrated polymorphic form of perindopril L-arginine invariably use a large excess of water or a crystallisation mixture with less than 1.0 molar equivalent of water, resulting in a low yield of the isolated hydrated form of perindopril L-arginine.

If the amount of water in the crystallisation solution exceeds 1.40 molar equivalents, calculated per perindopril L-arginine, the filtration time of the resulting suspension after 3 hours at the final crystallisation temperature is longer or the relative flow rate of the mother liquor is reduced, moreover the amount of solvent and antisolvent in the isolated wet filter cake is also higher.

If the amount of water in the process is not controlled and water is added to the crystallisation solution in larger quantities, e.g. more than 2.5 molar equivalents, calculated per perindopril L-arginine, after 2-3 hours of stirring at the final crystallisation temperature, a gel-like suspension is formed and the filtration time of the resulting suspension of the hydrated form of perindopril L-arginine is considerably prolonged, or the relative flow rate of the mother liquor is reduced. In this case, the isolated wet filter cake contains a significantly lower proportion of dry matter.

In the step of adding a seeding material, the hydrated form of perindopril L-arginine is added as a seeding material in an amount of 0.5-1.5% by weight relative to perindopril L-arginine.

In the process according to the invention, a stable suspension of the hydrated form of perindopril L-arginine with improved filtration properties is formed which, when stirred, retains unchanged filtration properties for a period of up to 3 hours after the suspension has been formed.

The crystallisation suspension of the hydrated form of perindopril L-arginine formed in the process according to the invention has a filtration time more than 10 times shorter compared to the processes described in the prior art, e.g. in patent EP 1989182 B1. For example, in an experiment where 20 g of perindopril was used, the filtration time of the suspension of the hydrated form of perindopril L-arginine obtained by the process according to the invention was 1 minute and the relative mother liquor flow rate was 6300 kg/h/m² (Example 1, given hereafter) and in an experiment where 1 kg of perindopril tert-butylamine was used, the filtration time of the suspension of the hydrated form of perindopril L-arginine obtained by the process according to the invention was 3 minutes, and the relative mother liquor flow rate was 5800 kg/h/m² (Example 3, given hereafter).

Therefore, the process according to the invention is suitable for efficient and easy application on an industrial scale.

The wet filter cake of the hydrated form of perindopril L-arginine, isolated by the process according to the invention, has a dry residue value after drying in the range of 50-70%. This means that 50-70% (w/w) of the hydrated form of perindopril L-arginine is present in the resulting wet filter cake, the remainder being the solvent and the antisolvent. A higher dry residue value after drying means less solvent and antisolvent in the final isolated wet filter cake and vice versa. Due to the lower amount of solvent and antisolvent in the wet cake isolated by the process according to the invention, the wet product has a shorter drying time.

The correlation between the amount of water in the crystallisation solution and the isolation properties, i.e. the filtration time of the suspension of the hydrated form of perindopril L-arginine and the dry residue after drying, is shown in Table 1 which is presented in the present patent application hereinafter.

The hydrated form of perindopril L-arginine obtained after drying the filter cake obtained by the process according to the invention has high chromatographic purity and adequate stability under storage conditions at room temperature. The resulting hydrated form of perindopril L-arginine is stable under normal storage conditions at room temperature.

The hydrated form of perindopril L-arginine obtained by the process according to the invention is physically stable, with no observed tendency in the X-ray diffraction profile of the sample, and is chemically stable, with no observed tendency of degradation in the chromatographic profile.

The following apparatus were used in the processes and examples described below:
X-ray diffraction was performed on a PANalytical - XPert Pro MPD apparatus.

The chromatographic analysis was carried out using a Hitachi Primaide system apparatus. The results were given as a percentage of peak area or as an area percentage of the total area of the integrated peaks - area %.

The water content was determined on a Metrohm Karl-Fisher titrator (Model: 794 Basic Titrino), sample weight ranging from 400 mg to 600 mg.

The correlation between the stirring time of the suspension of the hydrated form of perindopril L-arginine at a final crystallisation temperature of 25°C and the filtration time of the suspension is presented in Table 2 which is given in the present patent application hereinafter. The term stirring time, as used in the present application, means the stability time of the resulting suspension that is stirred.

Table 2 shows the correlation between the filtration time of the suspension at the final crystallisation temperature and the isolation properties of the product. Stirring the suspension of the hydrated form of perindopril L-arginine for more than 3 hours at the final crystallisation temperature has a negative impact on the filtration properties of the resulting suspension. If the suspension is stirred for more than 12 hours, the suspension becomes immiscible and has the properties of a gel.

When perindopril tert-butylamine is used as the starting material in the process according to the present invention, isolation of solid perindopril as an intermediate is not required.

The process according to the invention is advantageous in that it provides a simple and robust crystallisation process in which, by controlling and adjusting the appropriate amount of water in the crystallisation solution, a suspension of a hydrated form of perindopril L-arginine with excellent filtration properties is obtained, and which results in a high molar yield of conversion from perindopril tert-butylamine or perindopril to perindopril L-arginine.

The process according to the invention is cost-effective and suitable for being transferred to industrial scale for the routine production of a hydrated form of perindopril L-arginine.

The hydrated form of perindopril L-arginine obtained after drying the filter cake obtained by the process according to the invention is suitable for further use in the pharmaceutical industry, for example for the preparation of pharmaceutical dosage forms, preferably for the preparation of tablets.

The equivalents (equiv.) mentioned in the text of the present application are molar equivalents.

The following embodiments are given for the purpose of illustrating an embodiment of the invention and do not limit the invention in any way.

### Embodiments

### Process for preparing perindopril N-carboxy anhydride

Disodium hydrogen phosphate (43.1 g) was added to water (300 mL), the resulting mixture was stirred and heated at 30-35°C to obtain a clear solution. The clear solution was cooled to 25-30°C and dichloromethane (240 mL) and N-[(S)-1-carbethoxy-1-butyl]-(S)-alanine (30.0 g) were added while stirring and cooling to 15-20°C. A solution of triphosgene (16.2 g) dissolved in dichloromethane (60 mL) was slowly added to the reaction mixture at 15-20°C. After the addition, the mixture was stirred for 30 minutes. Pyridine (0.1 mL) was added to the reaction mixture and stirred for a further 1 hour. The organic phase was separated and washed first with 2N hydrochloric acid solution (150 mL) and then with water (150 mL). The organic phase was filtered and dichloromethane was distilled off under reduced pressure to give a pale-yellow oily residue, perindopril N-carboxy anhydride, weighing 39.3 g. The dry residue value after distillation of perindopril N-carboxy anhydride is 84%.

### Process for preparing perindopril

(2S,3aS,7aS)-octahydro-1H-indole-2-carboxylic acid (15.0 g), dichloromethane (DCM) (110 mL) and triethylamine (12.28 g) were added to a reaction flask and stirred for 10-15 minutes at room temperature. The perindopril N-carboxy anhydride oily residue with a dry matter content of 84% (30-7 g) - obtained by the above process - and dichloromethane (40 mL) were added slowly at room temperature. After the addition, the reaction mixture was stirred for 1 hour. Water (115 mL) was added and the reaction mixture was cooled to 15-20°C, the pH of the reaction mixture was adjusted to pH 5.6-5.8 using a 2N hydrochloric acid solution. After pH adjustment, the layers were separated and the aqueous layer was washed twice with dichloromethane (50 mL). The organic phases were combined and distilled at reduced pressure and at a temperature below 40°C to give the oily residue, pure (2S,3aS,7aS)-1-ethoxy-1-1-1-oxopentan-2-yl)-L-alanyl) octahydro-1H-indol-2-carboxylic acid (perindopril) weighing 31.7 g.

### Process for preparing crude perindopril tert-butylamine

To 31.7 g of the oily residue, containing pure (2S,3aS,7aS)-1-ethoxy-1-1-oxopentan-2-yl)-L-alanyl) octahydro-1H-indol-2-carboxylic acid obtained by the above process for preparing perindopril, ethyl acetate (460.0 g) was added and stirred for 10 minutes at 15-20°C. If necessary, filtering was carried out to remove undissolved particles. Tert-butylamine (7.14 g) was added and stirred for 30 minutes at 20°C. The resulting suspension was heated to reflux temperature and stirred until completely dissolved, but not more than 15 minutes. The resulting solution was then cooled to 20°C in a period of 3 hours and stirred for 2 hours at a final temperature of 20°C. The suspension was filtered and washed with ethyl acetate (45.0 g) to give a wet product, crude perindopril tert-butylamine, weighing 46.0 g. The dry residue value after drying of perindopril tert-butylamine is 79%.

### Recrystallisation of crude perindopril tert-butylamine

To a wet filter cake of perindopril tert-butylamine (46.0 g) obtained by the above process for preparing crude perindopril tert-butylamine, ethyl acetate (525.0 g) and 0.5 g of tert-butylamine were added and stirred for 10 min at 20°C. The suspension was heated to reflux temperature and stirred until completely dissolved, but not more than 15 minutes. The resulting solution was then cooled to 20°C in a period of 2 hours and stirred for 2 hours at a final temperature of 20°C. The suspension was filtered and washed with ethyl acetate (45.0 g). The resulting material, perindopril tert-butylamine, was dried in a dryer for 12 hours at 30°C and a pressure of 50 mbar. The weight of the dried perindopril tert-butylamine is 35.3 g.

### Crystallisation of perindopril

Perindopril tert-butylamine (30.0 g) obtained by the above process was added to a reaction vessel and water (75 g) and dichloromethane (100 g) were added. The mixture was cooled to 5°C and hydrochloric acid (20% solution) was added to adjust the pH to 4.0. It was stirred for 30 minutes and then left to stand to allow the phases to separate. The aqueous layer was washed with dichloromethane (100 g), stirred for 30 minutes and then stirring was discontinued for the phases to separate. The combined dichloromethane phases were evaporated under vacuum at 30°C. After evaporation, N-heptane (400 g) was added to the resulting oily residue and stirred at 20°C for 2 hours. The obtained material was filtered and dried for 12 hours at 40°C and a pressure of 50 mbar in a dryer to obtain 22.5 g of (2S,3aS,7aS)-1-ethoxy-1-1-oxopentan-2-yl)-L-alanyl)octahydro-1H-indole-2-carboxylic acid.

### Example 1

### Process for preparing a hydrated form of perindopril L-arginine from perindopril as a starting material

Perindopril obtained by the above process (20.0 g, calculated as per 100% perindopril content) was dissolved in ethanol (100 g) at 45°C. L-arginine (10.02 g, 1.06 equiv.) was added to the solution and stirred until completely dissolved. If necessary, the resulting solution (130.0 g) was filtered through a GF/B filter to obtain a completely clear solution. The solvent (83 g of ethanol solvent) was distilled off the resulting solution using a vacuum at 40°C. The partially evaporated solution weighing 47 g was cooled to 25°C. Ethyl acetate (320 g, water content < 0.1% by weight) was added to the solution at 25°C. The solution was stirred for a few minutes and the water content was measured and then adjusted to 1.15 equivalents, calculated with respect to perindopril L-arginine - the total amount of water in the crystallisation solution was 1.12 g. After adding water, the seeding material, the hydrated form of perindopril L-arginine (0.15 g), was added and the resulting suspension was stirred for 3 hours at 25°C. The isolation technique of vacuum filtration was used and the resulting wet filter cake was dried for 10 hours at 50°C and a pressure of 50 mbar.

The molar yield of the process was 92.3 %, the filtration time of the product, the hydrated form of perindopril L-arginine, was 1 minute, the relative mother liquor flow rate was 6300 kg/h/m², and the wet filter cake had a dry residue value after drying of 60%.

The resulting solid after drying was identified as the hydrated form of perindopril L-arginine with a water content of 3.3% by weight.

The sample was packed in double 100 µm polyethylene bags with silica gel and stored in an aluminium heat-sealed bag.

### Example 2

Process for preparing a hydrated form of perindopril (L)-arginine from perindopril tert-butylamine as a starting material.

Perindopril tert-butylamine (24.2 g, calculated to 100% perindopril tert-butylamine content) was dissolved in water (60.5 g) and dichloromethane (80.0 g) was added at 2°C. Hydrochloric acid solution (20% solution) was added dropwise to the reaction mixture until the pH reached 3.9. The layers were separated and the aqueous layer was washed with dichloromethane (80.0 g), and the organic phase was collected. The organic phases were combined and ethanol (242 g) was added to the organic phase and the solvent mixture was distilled off. On completion of distillation, a solution weighing 120 g was obtained, the water content in this solution had to be ≤ 0.35% by weight (water content less than 0.5 equiv. with respect to perindopril). If the criterion was not met, ethanol (60.5 g) was added and the solvent mixture distilled off (60.5 g), or the addition of ethanol was repeated (60.5 g) and then distilled off (60.5 g) until the water content in the solution weighing 120.0 g of ≤ 0.35% by weight was achieved. L-arginine (10.02 g, 1.06 molar equiv.) was added to the solution and stirred until completely dissolved at 45°C. If necessary, the solution was filtered through a GF/B filter to obtain a completely clear solution. The solvent was distilled off the resulting solution using vacuum evaporation at 40°C. The partially evaporated solution weighing 47 g was cooled to 25°C. Ethyl acetate (320 g, water content < 0.1% by weight) was added to the solution at 25°C. The resulting crystallisation solution of perindopril L-arginine was stirred for a few minutes, the water content was controlled and adjusted to 1.15 molar equivalents, calculated with respect to perindopril L-arginine - the total amount of water in the crystallisation mixture was 1.12 g. After adding water, the seeding material, the hydrated form of perindopril L-arginine (0.15 g), was added and the resulting suspension was stirred for 3 hours at the final crystallisation temperature of 25°C. The vacuum filtration was used as the isolation technique and the obtained wet filter cake was dried for 10 hours at 50°C and a pressure of 50 mbar.

The molar yield of the process was 91.4 %, the filtration time of the product, the hydrated form of perindopril L-arginine, was 1 minute, the relative mother liquor flow rate was 6200 kg/h/m², and the wet cake had a dry residue value after drying of 65%.

The obtained solid after drying was identified as the hydrated form of perindopril L-arginine with a water content of 3.4% by weight.

The sample was packed in double 100 µm polyethylene (PE) bags with silica gel and stored in an aluminium heat-sealed bag.

### The correlation between the amount of water in the crystallisation solution and the isolation (filtration) properties of the suspension of the hydrated form of perindopril L-arginine

Following the process described in Example 2 above, a hydrated form of perindopril L-arginine was prepared from perindopril tert-butylamine as a starting material, wherein various amounts or molar equivalents of water, calculated with respect to perindopril L-arginine, were added to the crystallisation solution during the process.

The results represented in Table 1 below show the correlation between the amount of water in the crystallisation solution of perindopril L-arginine and the isolation properties of the suspension of the hydrated form of perindopril L-arginine, resulting from the process. If the amount of water in the crystallisation solution exceeded 1.4 molar equivalents, calculated with respect to perindopril L-arginine, the filtration time of the suspension of the hydrated form of perindopril L-arginine resulting from the process was considerably longer and the amount of solvent and antisolvent in the isolated wet filter cake was also higher.

If the amount of water in the process was not controlled and water was added to the crystallisation solution of perindopril L-arginine in larger quantities, e.g. more than 2.5 molar equivalents, calculated with respect to perindopril L-arginine, after 2-3 hours of stirring the suspension of the hydrated form of perindopril L-arginine at the final crystallisation temperature, a gel-like suspension was formed and the filtration time of the resulting suspension was considerably prolonged.

Less than 1.0 molar equivalent of water in the crystallisation solution, calculated with respect to perindopril L-arginine, resulted in a lower yield of the hydrated form of perindopril L-arginine.

**Table 1:**

| Process according to example | Amount of water in crystallisation solution (molar equiv.) | Filtration time | Dry residue after drying (% by weight) |
|---|---|---|---|
| | | (min) | |
| Example 2 | 1.2 | 1 | 65 |
| Example 2 | 1.4 | 1 | 53 |
| Example 2 | 2.5 | 5 | 33 |
| Example 2 | 4.0 | 6 | 27 |

### The correlation between the stirring time of the suspension of the hydrated form of perindopril L-arginine at a final crystallisation temperature and the filtration time of the suspension

Following the process described in Example 2 above, a hydrated form of perindopril L-arginine was prepared from perindopril tert-butylamine as a starting material, wherein the stirring time of the suspension of the hydrated form of perindopril L-arginine formed in the process was changed at the final crystallisation temperature of 25°C.

Table 2 shows the correlation between the stirring time at the final crystallisation temperature and the filtration time of the suspension. Stirring longer than 3 hours at the final crystallisation temperature had a negative impact on the filtration properties. The suspension became immiscible and gel-like if stirred for more than 12 hours.

**Table 2:**

| Process according to example | Amount of water in crystallisation solution (molar equiv.) | Stirring time at final temperature (hours) | Filtration time | Dry residue after drying (% by weight) |
|---|---|---|---|---|
| | | | (min) | |
| Example 2 | 1.2 | 2 | 1 | 67 |
| Example 2 | 1.2 | 3 | 1 | 65 |
| Example 2 | 1.2 | 6 | 2 | 38 |
| Example 2 | 1.2 | 18 | 7 | 27 |

### Stability assays

### Stability assay under accelerated conditions - storage conditions at 40°C and 75% relative humidity (RH)

The hydrated form of perindopril L-arginine, prepared according to the process described in Example 2 above, was exposed to storage conditions at 40°C and 75% relative humidity for 6 months.

The hydrated form of perindopril L-arginine was found to be chemically and physically stable at storage conditions of 40°C and 75% relative humidity after 6 months with a chromatographic purity of more than 99.7%. The samples were packed in double polyethylene bags to prevent moisture absorption on the product and to control the water content of the packed sample to be below 4.0 % by weight. Silica gel was used as a drying agent. A precisely measured amount of water in the crystallisation solution ensures that the sample does not contain additional non-crystal-bound water after drying.

The hydrated form of perindopril L-arginine obtained by the process according to the invention is physically stable, with no observed tendency in the diffraction pattern of the sample, and chemically stable, with no observed degradation in the chromatographic profile.

The investigation of the stability of the samples or the observation of the degradation of the samples was mainly focused on the investigation of the tendency of two impurities: impurity B (Imp. B, 2S,3aS,7aS)-1-[(2S)-2-[[[(1S)-1-carboxybutyl]-amino]propanoyl]octahydro-1H-indole-2-carboxylic acid) and Impurity F (Imp. F, ethyl (2S)-2-[(3S,5aS,9aS,10aS)-3-methyl-1,4-dioxodecahydropyrazino-[1,2-a]indol-2(1H)-yl]pentanoate) according to the analytical procedure described in the European Pharmacopoeia PhEur 9.0. Impurity B and impurity F are typical impurities of perindopril degradation due to the hydrolytic mechanism, usually caused by exposure to water, temperature and time.

Table 3 presents the stability samples of the hydrated form of perindopril L-arginine prepared according to the process described in Example 2 above, wherein the amount of L-arginine in the salt formation reaction was varied.

The samples were packed in double 100 µm polyethylene bags with silica gel and stored in an aluminium heat-sealed bag to protect the samples against moisture ingress.

The parameter investigated was the amount of L-arginine added to the crystallisation solution, which influences the L-arginine content in the isolated material. The results show that a slight excess of L-arginine molar equivalent must be used for salt formation to effectively control the low level of impurity F when tested under storage conditions at 40°C and 75% relative humidity for 6 months. The diffraction pattern of the hydrated sample remains identical after 6 months at storage conditions of 40°C and 75% relative humidity compared to the initial sample.

**Table 3:**

| Process according to example | Amount of added L-arginine (molar equivalents) | Storage conditions at 40°C / 75% RH | HPLC purity (area %) | Imp. F (area %) | Imp. B (area. %) |
|---|---|---|---|---|---|
| Example 2 | 0.96 | t = 0 day | 99.95 | 0.01 | 0.03 |
| | | t = 6 months | 99.68 | 0.26 | 0.06 |
| Example 2 | 1.01 | t = 0 day | 99.95 | 0.01 | 0.03 |
| | | t = 6 months | 99.81 | 0.13 | 0.06 |
| Example 2 | 1.06 | t = 0 day | 99.95 | 0.01 | 0.03 |
| | | t = 6 months | 99.86 | 0.04 | 0.08 |

### Correlation between an increase in the amount of water and an increase in impurity B

The results presented in Table 4 show the correlation between stability under conditions at 40°C and 75% relative humidity. Table 4 shows that an increase in the water content in the storage of the material - the hydrated form of perindopril L-arginine - leads to an increase in the amount of impurity B. It is essential that the water content in the stored material is controlled to be below 4.0 % by weight during storage/storage conditions at 40°C and 75% relative humidity for 6 months, resulting in an impurity content at an appropriately low level.

The investigated material, the hydrated form of perindopril L-arginine, is hygroscopic when exposed to relative humidity > 40% at room temperature.

**Table 4:**

| Process according to example | Amount of added L-arginine | Water content (% by weight) | Storage conditions at 40°C / 75% RH | HPLC purity (area %) | Imp. B (area %) |
|---|---|---|---|---|---|
| | (molar equivalents) | | | | |
| Example 2 | 1.05 | 3.4 | t = 0 day | 99.95 | 0.01 |
| | | 3.4 | t = 6 months | 99.68 | 0.08 |
| Example 2 | 1.05 | 3.4 | t = 0 day | 99.95 | 0.01 |
| | | 4.1 | t = 6 months | 99.81 | 0.30 |
| Example 2 | 1.05 | 3.4 | t = 0 day | 99.95 | 0.01 |
| | | 5.8 | t = 6 months | 98.49 | 0.95 |

### Stability assay under stress conditions at 70°C and 40% relative humidity (RH)

### Correlation between an increase in the water content in the storage of the material - the hydrated form of perindopril L-arginine - and an increase in the amount of impurity B

Table 5 shows the results of stress conditions at a temperature of 70°C and 40% relative humidity. A comparison experiment with a water content of 5.4 % by weight after 14 days shows that impurity B (Imp. B) content increased up to 6 times faster compared to the sample in which the water content was controlled and no increase in the water content of the sample was observed after 14 days.

**Table 5:**

| Process according to example | Water content | Stress conditions at 70°C / 40% RH | HPLC purity (area %) | Imp. B (area %) |
|---|---|---|---|---|
| | (% by weight) | | | |
| Example 2 | 3.3 | t = 0 day | 99.95 | 0.01 |
| | 3.3 | t = 14 days | 99.85 | 0.06 |
| Example 2 | 6.8 | t = 0 day | 99.95 | 0.01 |
| | 5.4 | t = 14 days | 99.50 | 0.40 |

### Example 3

### Scale-up process based on the process described in Example 2

Perindopril tert-butylamine (1.0 kg, calculated with respect to 100% perindopril tert-butylamine content) was dissolved in water (2.5 kg) and dichloromethane (3.3 kg) at 2°C. A hydrochloric acid solution (20% solution) was slowly added to the reaction vessel until the pH 3.9 was reached. The layers were separated and the aqueous phase was washed with dichloromethane (3.3 kg), the organic phase was collected. Ethanol (10.0 kg) was added to the combined organic phase (perindopril formation yield is 99%) and the solvent mixture was distilled off. On completion of distillation, a solution weighing 5.0 kg was obtained, the water content of this solution had to be ≤ 0.35% by weight (water content less than 0.5 equiv. with respect to perindopril). If the criterion is not met, ethanol (2.5 kg) is added and the solvent mixture (2.5 kg) distilled off until the water content of ≤ 0.35% by weight is reached or the addition of ethanol is repeated (2.5 kg) and then distilled off (2.5 kg) until the water content in the solution weighing 5.0 kg of ≤ 0.35% by weight is achieved. L-arginine (0.414 kg, 1.06 equiv.) was added to the solution and stirred until completely dissolved at 45°C. If necessary, the solution was filtered through a GF/B filter to obtain a completely clear solution. The solvent was distilled off the resulting solution using a vacuum at 40°C. The partially evaporated solution weighing 1.94 kg was cooled to 25°C. Ethyl acetate (13.2 kg, water content < 0.1% by weight) was added to the solution at 25°C. The solution was stirred for a few minutes, the water content was adjusted to 1.15 molar equivalents, calculated with respect to equiv. of perindopril L-arginine - the total amount of water in the crystallisation mixture was 46.3 g. After having added water, the seeding material, the hydrated form of perindopril L-arginine (6.2 g), was added and the suspension was stirred for 3 hours at 25°C. The vacuum filtration was used as the isolation technique and the wet filter cake was dried in a dryer for 12 hours at 50°C and a pressure of 50 mbar.

The molar yield was 91.2 %, the filtration time of the product, the hydrated form of perindopril L-arginine, was 3 minutes, the relative mother liquor flow rate was 5800 kg/h/m², and the wet cake had a dry residue value after drying of 60%.

The resulting solid after drying was identified as the hydrated form of perindopril L-arginine with a water content of 3.3% by weight.

The sample was packed in double 100 µm polyethylene bags with silica gel and stored in an aluminium heat-sealed bag.

### Comparative example 4

The hydrated form of perindopril L-arginine was prepared according to the process described in US Patent 7846961 B2, Example 1.

Water (91.5 g), perindopril (20.0 g) and L-arginine (9.45 g) were introduced into a reaction vessel at room temperature while stirring. When a clear solution was formed, methylcyclohexane (38.4 g) was added and then dimethyl sulfoxide (287 g) was slowly added. The mixture was stirred until the temperature of the heterogeneous mixture stabilised at about 20.0°C, after which the mixture was vacuum filtered and the obtained solid was washed and dried.

The molar yield was 91.0%, the filtration time of the product, the hydrated form of perindopril L-arginine, was 12 min, the relative mother liquor flow rate was 440 kg/h/m², and the wet cake had a dry residue value after drying of 35%.

The resulting solid after drying was identified as the hydrated form of perindopril L-arginine with a water content of 3.4% by weight.

### Comparative example 5

The hydrated form of perindopril L-arginine was prepared according to the process described in CN 103172696, Example 4.

Perindopril (20.0 g), L-arginine (9.48 g) and water (30.0 mL) were introduced into a reaction vessel equipped with a water separator (Dean-Stark trap) at room temperature while stirring. Once a clear solution was formed, cyclohexane (100 mL) was added and the mixture was stirred at reflux temperature until no more water separation was observed - water separation was complete and water was no longer removed from the reaction mixture. The reactor temperature was cooled to 25°C and stirred for 1 hour. The suspension was filtered and the resulting wet cake was dried.

The molar yield was 88.0%, the filtration time of the product, the hydrated form of perindopril L-arginine, was 13 min, the relative mother liquor flow rate was 380 kg/h/m², and the wet cake had a dry residue value after drying of 47%.

The resulting solid after drying was identified as the hydrated form of perindopril L-arginine with a water content of 4.2% by weight.

It becomes apparent from the above examples that the filtration times of the product in Example 1 and Example 2 are more than 10 times shorter than the filtration times or relative mother liquor flows in Comparative Example 4 and Comparative Example 5.

## Claims

1. A process for preparing a hydrated form of perindopril L-arginine, **characterized in that** perindopril or a perindopril salt is used as a starting material and **in that** the process comprises:
- forming a crystallisation solution comprising perindopril L-arginine, wherein forming the crystallisation solution comprises at least:
- forming a perindopril solution, wherein ethanol is used as a solvent and ethyl acetate is used as an antisolvent, and wherein the perindopril solution formed contains less than 0.5 mol. equiv. of water with respect to perindopril,
- adding L-arginine to the perindopril solution,
to form a crystallisation solution comprising perindopril L-arginine,
- measuring the amount of water in the obtained crystallisation solution comprising perindopril L-arginine,
- adjusting the amount of water in the obtained crystallisation solution by adding such an amount of water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a suspension of the hydrated form of perindopril L-arginine, having a relative mother liquor flow rate in the range of 5500-6500 kg/h/m²,
- filtering the obtained suspension of the hydrated form of perindopril L-arginine,
to obtain a final product, a wet filter cake of the hydrated form of perindopril L-arginine, having a dry residue value after drying in the range of 50-70%.

2. The process according to claim 1, **characterized in that** the molar yield of the process is 85-92%, preferably 87-92%.

3. The process according to any one of claims 1 to 2, **characterized in that** perindopril or perindopril tert-butylamine is used as a starting material.

4. The process according to any one of claims 1 to 3, **characterized in that** in the step of adding a seeding material, the hydrated form of perindopril L-arginine is added as a seeding material in an amount of 0.5-1.5% by weight relative to perindopril L-arginine.

5. The process according to any one of claims 1 to 4, **characterized in that** perindopril is used as a starting material and that the process comprises:
- forming a crystallisation solution comprising perindopril L-arginine, wherein forming the crystallisation solution comprises:
- forming a perindopril solution by dissolving perindopril in the solvent ethanol, wherein the perindopril solution formed contains less than 0.5 mol. equiv. of water with respect to perindopril,
- adding L-arginine to the obtained solution in an amount of up to 6% of the molar excess of L-arginine, preferably 2-4% of the molar excess of L-arginine, with respect to perindopril,
- distilling the obtained solution to partially remove the solvent,
- adding the antisolvent ethyl acetate,
to form a crystallisation solution comprising perindopril L-arginine,
and then:
- measuring the amount of water in the obtained crystallisation solution comprising perindopril L-arginine,
- adjusting the amount of water in the obtained crystallisation solution by adding such an amount of water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a stable suspension of the hydrated form of perindopril L-arginine having relative mother liquor flows in the range of 5500-6500 kg/h/m² and
- filtering the obtained suspension,
to obtain a final product, a wet filter cake of the hydrated form of perindopril L-arginine, having a dry residue value after drying in the range of 50-70%, and the molar yield of the process is 85-92%.

6. The process for preparing a hydrated form of perindopril L-arginine according to any one of claims 1 to 4, **characterized in that** perindopril tert-butylamin is used as a starting material and that the process comprises:
- forming a crystallisation solution comprising perindopril L-arginine, wherein forming the crystallisation solution comprises:
- forming a perindopril solution, wherein the perindopril solution formed contains less than 0.5 mol. equiv. of water with respect to perindopril, comprising:
- disolving perindopril tert-butylamine in a solvent, preferably water and dichloromethane,
- adjusting the pH of the solution to a pH in the range of 3.8-4.2,
- separating the resulting phases and washing of the aqueous phase with dichloromethane,
- collecting the organic phase and adding the solvent ethanol to the organic phase,
- distilling off the mixture of solvents to a water content in the perindopril solution of less than 0.5 mol. equiv. of water with respect to perindopril,
- if needed, adding the solvent ethanol and distilling off the mixture of solvents until a water content in the perindopril solution of less than 0.5 mol. equiv. of water with respect to perindopril is achieved,
- adding L-arginine in an amount of up to 6% of the molar excess of L-arginine, preferably 2-4% of the molar excess of L-arginine, with respect to perindopril,
- distilling the obtained solution to partially remove the solvent,
- adding the antisolvent ethyl acetate,
to form a crystallisation solution comprising perindopril L-arginine,
and then:
- measuring the amount of water in the obtained crystallisation solution comprising perindopril L-arginine,
- adjusting the amount of water in the obtained crystallisation solution by adding such an amount of water to the crystallisation solution that the amount of water in the crystallisation solution is adjusted to 1.0-1.4 molar equivalents of water, preferably 1.1-1.3 molar equivalents of water, calculated with respect to perindopril L-arginine,
- adding a seeding material to the obtained crystallisation solution, wherein the seeding material is the hydrated form of perindopril L-arginine,
- forming a stable suspension of the hydrated form of perindopril L-arginine having a relative mother liquor flow in the range of 5500-6500 kg/h/m², and
- filtering the obtained suspension,
to obtain a final product, a wet filter cake of the hydrated form of perindopril L-arginine, having a dry residue value after drying in the range of 50-70%.
and the molar yield of the process after drying is 85-92%.

7. A wet filter cake of the hydrated form of perindopril L-arginine obtained by the process according to any one of claims 1 to 6, **characterised in that** it has a dry residue content after drying in the range of 50-70%.

## Patentansprüche

1. Verfahren zur Herstellung einer hydratisierten Form von Perindopril-L-Arginin, **dadurch gekennzeichnet, dass** Perindopril oder ein Perindoprilsalz als Ausgangsstoff verwendet wird und dass das Verfahren Folgendes umfasst:
- Bilden einer Kristallisationslösung, die Perindopril-L-Arginin umfasst, wobei das Bilden der Kristallisationslösung mindestens Folgendes umfasst:
- Bilden einer Perindopril-Lösung, wobei Ethanol als Lösungsmittel verwendet wird und Essigsäureethylester als Antilösungsmittel verwendet wird und wobei die gebildete Perindopril-Lösung weniger als 0,5 Moläquiv. Wasser, bezogen auf Perindopril, enthält,
- Zugeben von L-Arginin zu der Perindopril-Lösung,
unter Bildung einer Kristallisationslösung, die Perindopril-L-Arginin umfasst,
- Messen der Wassermenge in der erhaltenen Kristallisationslösung, die Perindopril-L-Arginin umfasst,
- Einstellen der Wassermenge in der erhaltenen Kristallisationslösung durch Zugeben einer solchen Wassermenge zu der Kristallisationslösung, dass die Wassermenge in der Kristallisationslösung auf 1,0-1,4 Moläquivalente Wasser, vorzugsweise 1,1-1,3 Moläquivalente Wasser, berechnet bezogen auf Perindopril-L-Arginin, eingestellt wird,
- Zugeben eines Impfmaterials zu der erhaltenen Kristallisationslösung, wobei es sich bei dem Impfmaterial um die hydratisierte Form von Perindopril-L-Arginin handelt,
- Bilden einer Suspension der hydratisierten Form von Perindopril-L-Arginin mit einer relativen Mutterlaugendurchflussrate im Bereich von 5500-6500 kg/h/m²;
- Filtrieren der erhaltenen Suspension der hydratisierten Form von Perindopril-L-Arginin,
unter Erhalt eines Endprodukts, eines feuchten Filterkuchens der hydratisierten Form von Perindopril-L-Arginin, mit einem Trockenrückstandswert nach Trocknung im Bereich von 50-70 %.

2. Verfahren nach Anspruch 1, **dadurch gekennzeichnet, dass** die molare Ausbeute des Verfahrens 85-92 %, vorzugsweise 87-92 %, beträgt.

3. Verfahren nach einem der Ansprüche 1 bis 2, **dadurch gekennzeichnet, dass** als Ausgangsstoff Perindopril oder Perindopril-tert-Butylamin verwendet wird.

4. Verfahren nach einem der Ansprüche 1 bis 3, **dadurch gekennzeichnet, dass** im Schritt des Zugebens eines Impfmaterials die hydratisierte Form von Perindopril-L-Arginin als Impfmaterial in einer Menge von 0,5-1,5 Gew.-%, bezogen auf Perindopril-L-Arginin, zugegeben wird.

5. Verfahren nach einem der Ansprüche 1 bis 4, **dadurch gekennzeichnet, dass** Perindopril als Ausgangsstoff verwendet wird und dass das Verfahren Folgendes umfasst:
- Bilden einer Kristallisationslösung, die Perindopril-L-Arginin umfasst, wobei das Bilden der Kristallisationslösung Folgendes umfasst:
- Bilden einer Perindopril-Lösung durch Lösen von Perindopril in dem Lösungsmittel Ethanol, wobei die gebildete Perindopril-Lösung weniger als 0,5 Moläquiv. Wasser, bezogen auf Perindopril, enthält,
- Zugeben von L-Arginin zu der erhaltenen Lösung in einer Menge von bis zu 6 % des molaren Überschusses an L-Arginin, vorzugsweise 2-4 % des molaren Überschusses an L-Arginin, bezogen auf Perindopril,
- Destillieren der erhaltenen Lösung zur teilweisen Entfernung des Lösungsmittels,
- Zugeben des Antilösungsmittels Essigsäureethylester,
unter Bildung einer Kristallisationslösung, die Perindopril-L-Arginin umfasst,
und dann:
- Messen der Wassermenge in der erhaltenen Kristallisationslösung, die Perindopril-L-Arginin umfasst,
- Einstellen der Wassermenge in der erhaltenen Kristallisationslösung durch Zugeben einer solchen Wassermenge zu der Kristallisationslösung, dass die Wassermenge in der Kristallisationslösung auf 1,0-1,4 Moläquivalente Wasser, vorzugsweise 1,1-1,3 Moläquivalente Wasser, berechnet bezogen auf Perindopril-L-Arginin, eingestellt wird,
- Zugeben eines Impfmaterials zu der erhaltenen Kristallisationslösung, wobei es sich bei dem Impfmaterial um die hydratisierte Form von Perindopril-L-Arginin handelt,
- Bilden einer stabilen Suspension der hydratisierten Form von Perindopril-L-Arginin mit relativen Mutterlaugendurchflussraten im Bereich von 5500-6500 kg/h/m² und
- Filtrieren der erhaltenen Suspension,
unter Erhalt eines Endprodukts, eines feuchten Filterkuchens der hydratisierten Form von Perindopril-L-Arginin, mit einem Trockenrückstandswert nach Trocknung im Bereich von 50-70 %, wobei die molare Ausbeute des Verfahrens 85-92 % beträgt.

6. Verfahren zur Herstellung einer hydratisierten Form von Perindopril-L-Arginin nach einem der Ansprüche 1 bis 4, **dadurch gekennzeichnet, dass** Perindopril-tert-Butylamin als Ausgangsstoff verwendet wird und dass das Verfahren Folgendes umfasst:
- Bilden einer Kristallisationslösung, die Perindopril-L-Arginin umfasst, wobei das Bilden der Kristallisationslösung Folgendes umfasst:
- Bilden einer Perindopril-Lösung, wobei die gebildete Perindopril-Lösung weniger als 0,5 Moläquiv. Wasser, bezogen auf Perindopril, enthält, umfassend:
- Lösen von Perindopril-tert.-Butylamin in einem Lösungsmittel, vorzugsweise Wasser und Dichlormethan,
- Einstellen des pH-Werts der Lösung auf einen pH-Wert im Bereich von 3,8-4,2,
- Trennen der resultierenden Phasen und Waschen der wässrigen Phase mit Dichlormethan,
- Sammeln der organischen Phase und Zugeben des Lösungsmittels Ethanol zu der organischen Phase;
- Abdestillieren der Lösungsmittelmischung bis zu einem Wassergehalt in der Perindopril-Lösung von weniger als 0,5 Moläquiv. Wasser, bezogen auf Perindopril,
- falls notwendig, Zugeben des Lösungsmittels Ethanol und Abdestillieren der Lösungsmittelmischung bis zum Erreichen eines Wassergehalts in der Perindopril-Lösung von weniger als 0,5 Moläquiv. Wasser, bezogen auf Perindopril,
- Zugeben von L-Arginin in einer Menge von bis zu 6 % des molaren Überschusses an L-Arginin, vorzugsweise 2-4 % des molaren Überschusses an L-Arginin, bezogen auf Perindopril,
- Destillieren der erhaltenen Lösung zur teilweisen Entfernung des Lösungsmittels,
- Zugeben des Antilösungsmittels Essigsäureethylester,
unter Bildung einer Kristallisationslösung, die Perindopril-L-Arginin umfasst,
und dann:
- Messen der Wassermenge in der erhaltenen Kristallisationslösung, die Perindopril-L-Arginin umfasst,
- Einstellen der Wassermenge in der erhaltenen Kristallisationslösung durch Zugeben einer solchen Wassermenge zu der Kristallisationslösung, dass die Wassermenge in der Kristallisationslösung auf 1,0-1,4 Moläquivalente Wasser, vorzugsweise 1,1-1,3 Moläquivalente Wasser, berechnet bezogen auf Perindopril-L-Arginin, eingestellt wird,
- Zugeben eines Impfmaterials zu der erhaltenen Kristallisationslösung, wobei es sich bei dem Impfmaterial um die hydratisierte Form von Perindopril-L-Arginin handelt,
- Bilden einer stabilen Suspension der hydratisierten Form von Perindopril-L-Arginin mit einer relativen Mutterlaugendurchflussrate im Bereich von 5500-6500 kg/h/m² und
- Filtrieren der erhaltenen Suspension,
unter Erhalt eines Endprodukts, eines feuchten Filterkuchens der hydratisierten Form von Perindopril-L-Arginin, mit einem Trockenrückstandswert nach Trocknung im Bereich von 50-70 %,
wobei die molare Ausbeute des Verfahrens nach dem Trocknen 85-92 % beträgt.

7. Feuchter Filterkuchen der hydratisierten Form von Perindopril-L-Arginin, erhalten nach dem Verfahren nach einem der Ansprüche 1 bis 6, **dadurch gekennzeichnet, dass** er nach dem Trocknen einen Trockenrückstandsgehalt im Bereich von 50-70 % aufweist.

## Revendications

1. Procédé de préparation d'une forme hydratée de périndopril L-arginine, **caractérisé en ce que** le périndopril ou un sel de périndopril est utilisé comme produit de départ et **en ce que** le procédé comprend:
- la formation d'une solution de cristallisation comprenant du périndopril L-arginine, dans lequel la formation de la solution de cristallisation comprend au moins:
- la formation d'une solution de périndopril, dans lequel de l'éthanol est utilisé comme solvant et de l'acétate d'éthyle est utilisé comme antisolvant, et dans lequel la solution de périndopril formée contient moins de 0,5 équiv. mol. d'eau par rapport au périndopril,
- l'ajout de L-arginine à la solution de périndopril,
pour former une solution de cristallisation comprenant du périndopril L-arginine,
- la mesure de la quantité d'eau dans la solution de cristallisation obtenue comprenant du périndopril L-arginine,
- l'ajustement de la quantité d'eau dans la solution de cristallisation obtenue en ajoutant à la solution de cristallisation une telle quantité d'eau que la quantité d'eau dans la solution de cristallisation soit ajustée à 1,0 à 1,4 équivalent molaire d'eau, de préférence 1,1 à 1,3 équivalent molaire d'eau, calculée par rapport au périndopril L-arginine,
- l'ajout d'un matériel d'ensemencement à la solution de cristallisation obtenue, dans lequel le matériel d'ensemencement est la forme hydratée de périndopril L-arginine,
- la formation d'une suspension de la forme hydratée de périndopril L-arginine, ayant un débit relatif de liqueur mère dans la plage de 5 500 à 6 500 kg/h/m²,
- la filtration de la suspension obtenue de la forme hydratée de périndopril L-arginine,
pour obtenir un produit final, un gâteau de filtration humide de la forme hydratée de périndopril L-arginine, ayant une valeur de résidu sec après séchage dans la plage de 50 à 70 %.

2. Procédé selon la revendication 1, **caractérisé en ce que** le rendement molaire du procédé est de 85 à 92 %, de préférence de 87 à 92 %.

3. Procédé selon l'une quelconque des revendications 1 à 2, **caractérisé en ce que** le périndopril ou le périndopril tert-butylamine est utilisé comme produit de départ.

4. Procédé selon l'une quelconque des revendications 1 à 3, **caractérisé en ce que** à l'étape d'ajout d'un matériel d'ensemencement, la forme hydratée de périndopril L-arginine est ajoutée comme matériel d'ensemencement en une quantité de 0,5 à 1,5 % en poids par rapport au périndopril L-arginine.

5. Procédé selon l'une quelconque des revendications 1 à 4, **caractérisé en ce que** le périndopril est utilisé comme produit de départ et **en ce que** le procédé comprend:
- la formation d'une solution de cristallisation comprenant du périndopril L-arginine, dans lequel la formation de la solution de cristallisation comprend:
- la formation d'une solution de périndopril par dissolution de périndopril dans le solvant éthanol,dans lequel la solution de périndopril formée contient moins de 0,5 équiv. mol. d'eau par rapport au périndopril,
- l'ajout de L-arginine à la solution obtenue en une quantité allant jusqu'à 6 % de l'excès molaire de L-arginine, de préférence de 2 à 4 % de l'excès molaire de L-arginine, par rapport au périndopril,
- la distillation de la solution obtenue pour éliminer partiellement le solvant,
- l'ajout de l'antisolvant acétate d'éthyle,
pour former une solution de cristallisation comprenant du périndopril L-arginine,
et ensuite:
- la mesure de la quantité d'eau dans la solution de cristallisation obtenue comprenant du périndopril L-arginine,
- l'ajustement de la quantité d'eau dans la solution de cristallisation obtenue en ajoutant à la solution de cristallisation une telle quantité d'eau que la quantité d'eau dans la solution de cristallisation soit ajustée à 1,0 à 1,4 équivalent molaire d'eau, de préférence 1,1 à 1,3 équivalent molaire d'eau, calculée par rapport au périndopril L-arginine,
- l'ajout d'un matériel d'ensemencement à la solution de cristallisation obtenue, dans lequel le matériel d'ensemencement est la forme hydratée de périndopril L-arginine,
- la formation d'une suspension stable de la forme hydratée de périndopril L-arginine ayant des débits relatifs de liqueur mère dans la plage de 5 500 à 6 500 kg/h/m² et
- la filtration de la suspension obtenue,
pour obtenir un produit final, un gâteau de filtration humide de la forme hydratée de périndopril L-arginine, ayant une valeur de résidu sec après séchage dans la plage de 50 à 70 %, et le rendement molaire du procédé est de 85 à 92 %.

6. Procédé de préparation d'une forme hydratée de périndopril L-arginine selon l'une quelconque des revendications 1 à 4, **caractérisé en ce que** le périndopril tert-butylamine est utilisé comme produit de départ et **en ce que** le procédé comprend:
- la formation d'une solution de cristallisation comprenant du périndopril L-arginine, dans lequel la formation de la solution de cristallisation comprend:
- la formation d'une solution de périndopril, dans lequel la solution de périndopril formée contient moins de 0,5 équiv. mol. d'eau par rapport au périndopril, comprenant:
- la dissolution du périndopril tert-butylamine dans un solvant, de préférence de l'eau et du dichlorométhane,
- l'ajustement du pH de la solution jusqu'à un pH dans la plage de 3,8 à 4,2,
- la séparation des phases résultantes et le lavage de la phase aqueuse avec du dichlorométhane,
- la collecte de la phase organique et l'ajout du solvant éthanol à la phase organique,
- la distillation du mélange de solvants jusqu'à une teneur en eau de la solution de périndopril inférieure à 0,5 équiv. mol. d'eau par rapport au périndopril,
- si nécessaire, l'ajout du solvant éthanol et la distillation du mélange de solvants jusqu'à ce que la teneur en eau de la solution de périndopril de moins de 0,5 équiv. mol. d'eau par rapport au périndopril soit obtenue,
- l'ajout de L-arginine en une quantité allant jusqu'à 6 % de l'excès molaire de L-arginine, de préférence de 2 à 4 % de l'excès molaire de L-arginine, par rapport au périndopril,
- la distillation de la solution obtenue pour éliminer partiellement le solvant,
- l'ajout de l'antisolvant acétate d'éthyle,
pour former une solution de cristallisation comprenant du périndopril L-arginine,
et ensuite:
- la mesure de la quantité d'eau dans la solution de cristallisation obtenue comprenant du périndopril L-arginine,
- l'ajustement de la quantité d'eau dans la solution de cristallisation obtenue en ajoutant à la solution de cristallisation une telle quantité d'eau que la quantité d'eau dans la solution de cristallisation soit ajustée à 1,0 à 1,4 équivalent molaire d'eau, de préférence 1,1 à 1,3 équivalent molaire d'eau, calculée par rapport au périndopril L-arginine,
- l'ajout d'un matériel d'ensemencement à la solution de cristallisation obtenue, dans lequel le matériel d'ensemencement est la forme hydratée de périndopril L-arginine,
- la formation d'une suspension stable de la forme hydratée de périndopril L-arginine ayant un débit relatif de liqueur mère dans la plage de 5 500 à 6 500 kg/h/m² et
- la filtration de la suspension obtenue,
pour obtenir un produit final, un gâteau de filtration humide de la forme hydratée de périndopril L-arginine, ayant une valeur de résidu sec après séchage dans la plage de 50 à 70 %,
et le rendement molaire du procédé après séchage est 85 à 92 %.

7. Gâteau de filtration humide de la forme hydratée de périndopril L-arginine obtenu par le procédé selon l'une quelconque des revendications 1 à 6, **caractérisé en ce qu'**il a une teneur en résidu sec après séchage dans la plage de 50 à 70 %.
